# EUROPÄISCHE PATENTSCHRIFT

(11) **EP 1 580 172 B1**
(45) Veröffentlichungstag und Bekanntmachung des Hinweises auf die Patenterteilung: **27.01.2010**
(21) Anmeldenummer: 05001612.0
(22) Anmeldetag: 27.01.2005
(51) Int. Cl.: C03C 3/16, C03C 3/062, C03C 10/00, C03C 12/00

(54) **Antimikrobiell brechzahlangepasstes Phosphatglas**
Anti-microbial, refractive index- adapted phosphate glass
anti-microbiel, indice de refraction-adapte verre phosphate

(30) Priorität: 08.03.2004 DE 102004011520
(43) Veröffentlichungstag der Anmeldung: 28.09.2005
(73) Patentinhaber: Schott AG, 55122 Mainz (DE)
(72) Erfinder: Seneschal, Karine Dr., 55131 Mainz (DE); Zimmer, José Dr., 55218 Ingelheim (DE); Fechner, Jörg Hinrich Dr., 55118 Mainz (DE); Schreder, Bianca Dr., 60322 Frankfurt (DE)
(74) Vertreter: Sawodny, Michael-Wolfgang

(56) Entgegenhaltungen:
- EP-A- 1 116 700
- WO-A-02/090278
- WO-A-2004/076371
- DE-A1- 10 241 495
- ES-A1- 2 190 732
- GB-A- 2 037 735
- JP-A- 7 300 339
- JP-A- 8 048 539
- US-A1- 5 290 544

## Beschreibung

Die Erfindung betrifft antimikrobielle Gläsern, Glaskeramiken, insbesondere Glasspulver und Glaskeramikpulver, Glasfasern, Glasgranulate, Glaskugeln auf der Basis von weitgehend alkalifreien Phosphatgläsern, die eine antimikrobielle Wirkung aufweisen

In der US 5 290 544 werden wasserlösliche Gläser für die Anwendung in kosmetischen Produkten mit sehr geringen SiO₂ und sehr hohen B₂O₃ beziehungsweise hohen P₂O₅ -Gehalten beschrieben. Die Gläser weisen Silberkonzentrationen < 0,5 Gew.-% auf. Diese Gläser besitzen eine extrem niedrige hydrolytische Beständigkeit und haben den Nachteil, sich in Wasser vollständig aufzulösen. Die antibakterielle Wirkung in diesen Gläsern wird durch die freiwerdenden Ag- und/oder Cu-lonen bewirkt.

In der US 6 143 318 werden silberhaltige Phosphatgläser beschrieben, die als antimikrobielles Material für die Wundinfektionsbehandlung Kombinationen aus Cu, Ag und Zn verwenden. Der Nachteil dieser Gläser ist die niedrige hydrolytische Beständigkeit, die sich darin ausdrückt, dass die Gläser vollständig wasserlöslich sind. Diese Gläser enthalten kein Al₂O₃, und/oder SiO₂ , die dazu dienen, die hydrolytische Beständigkeit einzustellen. Weiterhin ist die Konzentration von Na₂O mit 34 Mol-% sehr hoch. Dies bedingt, dass die Reaktivität des Glases sehr hoch ist und es sich relativ schnell komplett auflöst.

Phosphat- beziehungsweise Borophosphatgläser, die kein Titan aufweisen sind auch aus nachfolgenden Schriften
JP-A-2001-247333
JP-A 2001-247335
JP-A 2001-247336
JP-A 2001-247337
JP-A 92338129
bekannt geworden

JP-A-2001-247333 beschreibt eine Glasfaser die in einem späteren Prozessschritt mit Ag₂O antimikrobiell ausgerüstet wird.

JP-A 2001-247336, JP-A 2001-247335 beschreiben ebenfalls eine Glaszusammensetzung, die in einem nachgeschalteten Prozeßschritt durch Ag₂O antimikrobiell ausgerüstet wird

Die nachträgliche Zugabe von Ag₂O führt dazu, dass Kompositmaterialien ausgebildet werden, bei denen Silber bzw. Silberagglomerate an der Oberfläche der Glasphase angelagert werden, so dass keine homogene Verteilung des Silbers vorliegt.

In JP 92338129 wird ein lösliches Glas beschrieben, das frei von Alkalien und Aluminium sein kann. Dieses Glas erzielt seine antimikrobielle Wirkung durch den Zusatz von Silber.

Glaszusammensetzungen, die TiO₂ enthalten sind aus nachfolgenden Schriften bekannt geworden:
JP-A-2002-012442
JP-A-96048539
EP-A-141580
JP-A-2000-327369

Die aus den Schriften EP-A-141580 und JP-A-2000-327369 bekannten Gläser haben einen Phosphorgehalt niedriger als 45 Gew.-%.

Die aus den Schriften JP-A-2002-012442 und JP-A-96048539 bekannten Gläser haben einen Alkaliengehalt größer 0,39 Gew.-%, was bei einer Verwendung in Polymeren wie beispielsweise Polycarbonat den Nachteil hat, dass derartige Alkalienkonzentrationen zu einem Kettenbruch führen können und damit das Polymere degeneriert. In der Regel tritt des weiteren eine unerwünschte Verfärbung der Polymere auf.

Die JP 07 300339 A beschreibt eine antimikrobielle Glaszusammensetzung, umfassend 40-55 Mol% P₂O₅, 30-60 Mol % CaO + MgO, 0-4 Mol% SiO₂ + Al₂O₃, 0-15 Mol% Li₂O + B₂O₃, 0-5 Mol% Na₂O + K₂O, 0-30 Mol% ZnO + BaO, wobei CaO + MgO + ZnO + BaO ingesamt 30-60 Mol% darstellen, 0-5 Mol% PbO und 0,03-5 Mol% Ag₂O.

Aufgabe der Erfindung ist es, eine antimikrobielle Glaszusammensetzung anzugeben, die anderen Stoffen, beispielsweise Polymeren zugegeben werden kann wobei der Brechungsindex der Glaszusammensetzung einstellbar ist, um durch Brechzahlanpassung möglichst transparente Stoffe, insbesondere Polymere zu ermöglichen. Bevorzugt soll zudem die UV-Kante beziehungsweise die Transmission der Gläser einstellbar sein. Die Gläser sollen eine antimikrobielle Wirkung bei relativ hoher chemische Beständigkeit sowie hoher Reaktivität aufweisen. Insbesondere soll die Glaszusammensetzung hoch antimikrobiell sein und als Zuschlagstoffe auch für Polymere, wie beispielsweise Polycarbonaten geeignet sein. Des weiteren soll keine Vergilbung der Polymeren während des Herstellprozesses als auch in der Verwendung sowie keine nachtelligen mechanischen Effekte wie z.B. Versprödung auftreten. Besonders vorteilhaft ist es, wenn auch die chemische Beständigkeit eingestellt werden kann.

Erfindungsgemäß wird die Aufgabe durch die nachfolgenden, antimikrobiellen Glaszusammensetzungen (in Gew.-% auf Oxidbasis) gelöst, die auch Basis für Glaskeramiken sein können.

| | |
|---|---|
| P₂O₅ | >45- 90 Gew.-% |
| B₂O₃ | zwischen 1 und 45 Gew.-% |
| SiO₂ | 0 - 40 Gew.-% |
| Al₂O₃ | 0 -20 Gew.-% |
| SO₃ | 0 - 30 Gew.-% |
| Li₂O | 0 -0,1 Gew.-% |
| Na₂O | 0 -0,1 Gew.-% |
| K₂O | 0 -0,1 Gew.-% |
| CaO | 0 - 40 Gew.-% |
| MgO | 0 - 40 Gew.-% |
| SrO | 0 - 15 Gew.-% |
| BaO | 0 - 40 Gew.-% |
| ZnO | 0 - 40 Gew.-% |
| Ag₂O | 0 - 5 Gew.-% |
| CuO | 0 - 15 Gew.-% |
| Cr₂O₃ | 0 - 10 Gew.-% |
| J | 0 - 10 Gew.-% |
| TeO₂ | 0 - 10 Gew.-% |
| GeO₂ | 0 - 10 Gew.-% |
| TiO₂ | 0 - 10 Gew.-% |
| ZrO₂ | 0 - 10 Gew.-% |
| La₂O₃ | 0 - 10 Gew.-% |
| Nb₂O₃ | 0 - 5 Gew.-% |
| CeO₂ | 0 - 5 Gew.-% |
| Fe₂O₃ | 0 - 5 Gew.-% |
| WO₃ | 0 - 5 Gew.-% |
| Bl₂O₃ | 0 - 5 Gew.-% |
| MoO₃ | 0 - 5 Gew.-% |

wobei die Summe aus TiO₂ + ZrO₂ + BaO + La₂O₃ + Cr₂O₃ + Nb₂O₃ im Bereich 0,01- 40 Gew.%, bevorzugt im Bereich 0,1 - 40 Gew.-%, liegt und die Summe aus Ag₂O + ZnO + CuO + Cr₂O₃ + J + TeO₂ und GeO₂ im Bereich 0,1 - 40 Gew.-% liegt und die Zusammensetzung bis auf Verunreinigungen frei von Sn ist, wobei Ag₂O im Bereich 0,1 Gew.-% < Ag₂O ≤ 3 Gew,-% liegt und der CuO-Gehalt im Bereich 0,1 Gew.-% < CuO ≤ 10 Gew.-% liegt..

Dadurch, dass das Glas bis auf Verunreinigungen frei von Sn ist, wird verhindert, dass Ag⁺ zu Ag⁰ reduziert wird und so eine unerwünschte Verfärbung des Glases auftritt, falls Ag im Glas enthalten ist.

In einer bevorzugten Ausführungsform liegt die Summe aus Na₂O und K ₂O und Li₂O im Bereich 0 - 0,3 Gew.-%.

U iter antimikrobieller Wirksamkeit wird hier eine blozide bzw. biostatische Wirkung gegen Bakterien, Pilze, Algen, Hefen, Viren etc. verstanden.

In einer fortgebildeten Ausführungsform ist der Ag-Gehalt im Bereich 0,3 Gew.-% < Ag₂O ≤ 2 Gew.-%. Um eine besonders starke antimikrobielle Wirkung zu erzielen ist der Gehalt von Ag₂O grösser als 0,1 Gew.-%, bevorzugt grösser als 0,3 Gew.-%, insbesondere bevorzugt grösser 1 als Gew.-% .

Eine Silberkonzentration von ≤ 3 Gew.% ist besonders bevorzugt, wenn eine starke antimikrobielle Wirksamkeit bei gleichzeitig geringer bzw. keiner Verfärbung der Gläser gefordert ist. Obwohl durch die weitgehende Sn-Freiheit des antimikrobiellen Glases eine Reduktion von Ag⁺ zu Ag⁰ für Silberkonzentrationen ≤ 3 Gew.-% verhindert wird, kann auch bei Sn-freien Gläsern für Ag-Gehalte größer 3 Gew.-% eine Verfärbung auftreten.

Eine Verfärbung auch bei Ag-Gehalten größer 3 Gew.-% kann bei Sn-freien Gläsern gemäß der Erfindung durch den Zusatz von Edelmetalloxiden wie Au₂O₃, PtO₂, PdO₂ verhindert werden, da diese Edelmetalloxide die Reduktion von Ag verhindern. Bevorzugt liegt der Gehalt an Edelmetalloxiden im Bereich 0,001 bis 100 ppm, bevorzugt im Bereich 0,01 bis 10 ppm.

Alternativ oder zusätzlich kann durch Zugabe von einem oder mehreren Ionen seltener Erde wie beispielsweise Ce³⁺, Pr³⁺, Nd³⁺, Pm³⁺, Eu³⁺, Gd³⁺,Tb³⁺, Dy³⁺, Ho³⁺, Er³⁺, Tm³⁺, Yb³⁺ oder Metallionen wie Ti, V, Cr, Mn Fe, Co, Ni, Cu, die Verfärbung von Ag₂O auch bei Gehalten größer 3 Gew.-% kompensiert werden, weil komplementäre Farbeffekte ausgebildet werden.

In einer bevorzugten Ausführungsform beträgt der ZnO-Gehalt > 5 Gew.% ZnO zur Erzielung der antimikrobiellen Wirkung. Insbesondere falls wenig Silber in der Glaszusammensetzung vorhanden ist, ist der ZnO-Gehalt bevorzugt > 10 Gew.-Noch bevorzugter ist der ZnO-Gehalt > 20 Gew.-%. Dies ist insbesondere dann bevorzugt, wenn eine antimikrobielle Wirkung des Glases erreicht werden soll und das Glas einen geringen Silbergehalt aufweist oder bis auf Verunreinigungen frei Silber ist, um eine Verfärbung des Glases sicher zu verhindern.

In einer fortgebildeten Ausführungsform liegt der Cu-Gehalt im Bereich 0,5 Gew.-% < CuO ≤ 8 Gew.-%. Um eine hinreichend antimikrobielle Wirkung insbesonders gegen Pilze zu erzielen, ist der Gehalt von CuO grösser als 0,1 Gew.-% , bevorzug grösser 0,5 Gew.-%, noch bevorzugter grösser 1 Gew.-%, am bevorzugsten grösser 2 Gew.-%.

In einer besonders bevorzugten Ausführungsform der Erfindung enthält die Glaszusammensetzung sowohl Cu- wie auch Ag-lonen. Durch die Kombination von Cu und Ag wird eine synergistische antimikrobielle Wirkung erzielt. Durch das gezielte Einstellen der Gehalte an Ag und Cu-Ionen in der Glaszusammensetzung können insbesondere die bakterizide und die fungizide Wirkung des Glases eingestellt werden.

Cu wirkt besonders gut gegen Pilze, d.h. Kupfer hat eine hohe fungizide Wirkung. In Kombination mit Ag wird die fungizide Wirkung erhöht.

In einer fortgebildeten Ausführungsform ist die Summe der Cu-Gehalt und der Silber-Gehalt im Bereich 0,1 Gew.-% < CuO + Ag₂O ≤ 10 Gew.-%, besonders bevorzugt 0,3 Gew.-% < CuO + Ag₂O ≤ 8 Gew.-% .

Durch die Zugabe von Jod wird eine wundheilende und desinfizierende Wirkung erzielt.

Chrom wird in Anwendungsgebieten verwendet, in denen keine toxikologische Unbedenklichkeit gefordert wird und eine hohe antimikrobielle Wirkung erwünscht ist.

In einer fortgebildeten Ausführungsform ist der Cr-Gehalt im Bereich 0,3 Gew.-% < Cr₂O₃ ≤ 7 Gew.-%, besonders bevorzugt ist: 3 Gew.-% < Cr₂O₃ ≤ 5 Gew.-%. Um eine hinreichend antimikrobielle Wirkung zu erzielen, ist es vorteilhaft, wenn der Gehalt von Cr₂O₃ grösser als 0,3 Gew.-%, bevorzugt grösser 1 Gew.-%, am bevorzugsten grösser 2 Gew.-% ist. Eine Chromkonzentration von ≤ 5 Gew.-% ist besonders dann bevorzugt, wenn eine starke antimikrobielle Wirksamkeit bei gleichzeitig geringer bzw. keiner Verfärbung der Gläser gefordert ist.

Das erfindungsgemäße Glas des Glaspulvers enthält P₂O₅ als Netzwerkbildner, wobei der Vernetzungsgrad unter anderem durch die Schmelzparameter beeinflusst und die Schmelzbarkeit reduziert werden kann.

Wenn der P₂O₅-Gehalt geringer als 40 Gew.-% ist, ist die Glasstabilität gegen kristallisation zu gering. Wenn der P₂O₅-Gehalt grösser als 90 Gew.-% ist, wird die Korrossion des Tiegelmaterials in dem die Glaszusammensetzung erschmolzen wird, zu hoch. Außerdem ist bei hohen Gehalten an P₂O₅, über 80 Gew.-%, die chemische Beständigkeit der Glaszusammensetzung zu niedrig.

Bevorzugt liegt daher der Gehalt von P₂O₅ zwischen 45 und 80 Gew.-%, insbesondere zwischen 60 und 75 Gew.-%.

Das Glas kann B₂O₃ als weiteren Netzwerkbildner enthalten, um die Reaktivität zu erhöhen. Der B₂O₃ -Gehalt übersteigt nicht 60 Gew.-%, um eine ausreichende chemische Beständigkeit zu gewährleisten. Der B₂O₃-Gehalt liegt erfindungsgemäß zwischen 1 und 45 Gew.-%.

Das Glas kann SiO₂ als weitere Netzwerkbildner enthalten um die chemische Beständigkeit zu erhöhen. Dabei sollte der SiO₂ -Gehalt nicht höher als 40 Gew.-% sein, da sonst die Beständigkeit gegen Kristallisation zu gering ist und die chemische Beständigkeit zu hoch. Bevorzugt liegt der SiO₂ - Gehalt zwischen 0 und 30 Gew.-% , besonders bevorzugt zwischen 1 und 15 Gew.-%.

In einer besonders bevorzugten Ausführungsform der Erfindung ist die Glaszusammensetzung weitgehend frei von Al₂O₃. In einer besonders bevorzugten Ausführungsform ist der Gehalt von Al₂O₃ geringer als 1 Gew.-%

Al₂O₃ ist ein intermediäres Oxid, das die Konnektivität des Glasnetzwerkes verstärkt. Wenn Al₂O₃ in zu hohen Konzentrationen vorliegt, insbesondere größer als 10 Gew.-%, ist die chemische Beständigkeit der vorliegenden Gläser zu hoch. Für derartige Al-Gehalte wird eine zu niedrige Rate für die Ionenfreisetzung insbesondere von Ag oder Zn beobachtet und die antimikrobielle Wirkung ist sehr gering.

Des weiteren ist die Freisetzung von Al nachteilig für die Gesundheit. Beispielsweise können durch die Freisetzungen Krankheiten wie beispielsweise die Alzheimer-Krankheit bewirkt werden. Deswegen ist es von besonderem Vorteil, wenn das Glas weitgehend aluminiumfrei ist. Unter weitgehend aluminiumfrei wird in vorliegender Anmeldung ein Al₂O₃-Gehalt geringer als 1 Gew.-% verstanden.

Der Al₂O₃-Gehalt der erfindungsgemäßen Zusammensetzung beträgt gemäß der vorliegenden Erfindung höchstens 10 Gew.-%. Bevorzugt liegt der Al₂O₃-Gehalt unter 5 Gew.-%, besonders bevorzugt ist das Glas weitgehend aluminiumfrei.

Die in der erfindungsgemäßen Glaszusammensetzung enthaltenen Erdalkalioxide tragen zum Aufbau des Glasnetzwerkes bei. Durch den Anteil an Erdalkalioxiden in der Glaszusammensetzung kann die gewünschte Reaktivität des Glases eingestellt werden. Bevorzugt ist ein Bereich, bei dem die Summe aus MgO + CaO + SrO + BaO zwischen 2 Gew.-% und 40 Gew.-% liegt, da ein Gehalt MgO + CaO + SrO + BaO > 40 Gew.-% zu einer zu hohen chemischen Beständigkeit führt und zu einer verminderten Reaktivität des Glases. Wenn der Gehalt MgO + CaO + SrO + BaO < 2 Gew.-% ist, ist die chemische Beständigkeit zu gering und die Wasserlössigkeit zu hoch. Bevorzugt liegt daher die Summe der Erdalkalioxide im Bereich 4- 35 Gew.-%, bevorzugt im Bereich 5-35 Gew.-%.
In einer besonders bevorzugten Ausführungsform liegt die Summe der Gehalte an TiO₂ und ZrO₂ und BaO und La₂O₃ und Nb₂O₃ im Bereich 0,1- 30 Gew.-%. Die Zugabe derartiger Stoffe ermöglicht, wie oben beschrieben, die Einstellung des Brechungsindexes der antimikrobiellen Glaszusammensetzung. Damit ist es möglich das Glasmaterial mit antimikrobieller Wirkung an z.B. die Brechzahl eines Polymeren anzupassen, so dass auch die Mischung Polymer/antimikrobielles Glaspulver transparent ist. Außerdem kann eine Eintrübung vermieden werden. Eine besonders gute Einstellbarkeit des Brechungsindex ergibt sich, wenn das antimikrobielle Glas BaO enthält. Eine besonders gute Einstellbarkeit wird erzielt, wenn die Summe TiO₂ und ZrO₂ und BaO und La₂O₃ und Nb₂O₃ größer als 1 Gew.-% ist, bevorzugt größer als 5 Gew.-%.

Eine Brechzahlanpassung ist insbesondere von Vorteil wenn, der Glaszusatz in Farben und Lacken verwandt wird. Durch den erfindungsgemäßen Zusatz kann eine Eintrübung vermieden werden. Durch die Einstellung des Brechungsindexes des Zusatzes an den der Farben und Lacke wird der Farbton der Farbe durch den antimikrobiellen Zusatz nicht beeinträchtigt .

Durch den Zusatz von TiO₂ und ZrO₂ und BaO und La₂O₃ und Nb₂O₃ kann nicht nur der Brechungsindex in einem Volumenmaterial angepasst werden, sondern auch in einer Glasoberfläche, die eine antimikrobielle Wiorkung aufweist.

Wenn die Summe der Gehalt an TiO₂ und ZrO₂ und BaO und La₂O₃ und Nb₂O₃ größer als 40 Gew.-%, insbesondere größer als 30 Gew.-% ist, nimmt die Kristallisationsbeständigkeit des Glases zu stark ab und die chemische Beständigkeit des Glases wird sehr hoch, so dass antimikrobielle lonen wie Silber, Kupfer nur in geringen Menge freigesetzt werden und nur eine sehr geringe antimikrobielle Wirkung erzielt wird.

Ein weiterer Vorteil der erfindungsgemäßen Glaszusammensetzung ist, dass durch die Zugabe der Metallionen wie Ti, Ce, Fe, W, Bi, Mo, Nb UV-Licht absorbiert wird. Des weiteren kann durch den Zusatz von lonen wie Ti, Ce, Fe, W, Bi, Mo, Nb, auch die Vergilbung und/ oder die Sprödigkeit die aufgrund von UV Strahlung in Polymeren auftritt, verringert und sogar ganz verhindert werden.

Durch Zugabe nur einer der oben genannten Metallionen wird eine besonders effektive UV-Blockung erreicht.

Die Summe der Gehalte aus TiO₂ + CeO₂ + Fe₂O₃ + WO₃ + Bi₂O₃ MoO₃ + Nb₂O₃ ist in den erfindungsgemäßen Glaszusammensetzungen kleiner 20 Gew.-%. Bei Gehalten größer 20 Gew.-% ist die chemische Beständigkeit zu hoch und die Reaktivität des Glases zu gering, um eine ausreichende Menge antimikrobieller Ionen freizusetzen. Bevorzugt ist die Summe TiO₂ + CeO₂ + Fe₂O₃ + WO₃ + Bi₂O₃ + MoO₃ + Nb₂O₃ + <15 Gew.-% und > 0,1 Gew.-%, bevorzugter < 10 Gew.-% und > 0,1 Gew.-%. Durch den Anteil an Metalloxiden in der Glaszusammensetzung wie beispielsweise Ti kann eine gewünschte UV-Absorption eingestellt werden.

Bevorzugt liegen die Gehalte der neben Ti zugegebenen Metalloxide für ein oder mehrere lonen in nachfolgenden Zusammensetzungsbereichen:

| | | |
|---|---|---|
| CeO₂ | 0-1 | Gew.-% |
| Fe₂O₃ | 0-1 | Gew.-% |
| WO₃ | 0-3 | Gew.-% |
| Bi₂O₃ | 0-3 | Gew.-% |
| MoO₃ | 0-3 | Gew.-% |
| Nb₂O₃ | 0-3 | Gew.-% |

Ein weiterer Vorteil der erfindungsgemäßen weitegehend alkalifreien antimikrobiellen Zusammensetzungen gemäß der Erfindung ist, dass Pulver aus derartige Glas- bzw. Glaszusammensetzungen einen Vorteil bei Anwendung in bestimmten Kunststoffen oder Lacken umfassend Polymere haben, da die Polymerkette nicht aufgebrochen und damit der Polymerwerkstoff nicht lokal zerstört wird. Hierdurch wird sichergestellt, dass die mechanischen und optischen Eigenschaften des Polymerwerkstoffes nicht nachhaltig beeinträchtigt werden.

Insbesondere werden die Polymerketten, zum Beispiel in Polycarbonaten, nicht angegriffen, so dass die mechanischen und optischen Eigenschaften von Polycarbonaten durch die erfindungsgemäßen Phosphatglaspulver als Zuschlagstoffe nicht nachteilig beeinflusst werden.

Aufgrund des hohen Phosphorgehaltes können die erfindungsgemäßen Phosphatgläser, Glaspulver, Glaskeramiken oder Glaskeramikpulver, Glasflakes, Glaskugel, Hohlkugel neben der bioziden Wirkung durch Ionenaustausch bzw. lonenfreisetzung auch eine bioaktive Wirkung aufweisen. Die erfindungsgemäßen Phosphatgläser, Glaskeramiken, Glaspulver, Glaskeramikpulver, Glasflakes, Glaskugel, Hohlkugel sind daher besonders biokompatibel, d.h. besonders verträglich mit Körpergewebe.

In einer bevorzugten Ausführungsform kann der Schwermetallgehalt durch den vollständigen oder teilweisen Ersatz beispielsweise von Zn bevorzugt durch Ca, aber auch Mg verringert werden. Derartige Substanzen gewährleisten eine gute Umweltverträglichkeit.

Bei den erfindungsgemäßen Gläsern , Glaspulvern, Glaskeramiken oder Glaskeramikpulvern, Glasflakes, Glaskugel, Hohlkugel werden durch Reaktionen an der Glasoberfläche bzw. partielle Auflösung des Glases lonen ausgetauscht bzw. freigesetzt. Die antimikrobielle Wirkung beruht somit unter anderem auf einer Freisetzung von Ionen, insbesonders Silber-lonen. Die antimikrobielle Wirkung durch Ionenaustausch bzw. -freisetzung beeinträchtigen das Zellwachstum.

Neben der Abgabe spielt die in die Systeme eingebrachte antimikrobielle Glasoberfläche auch eine Rolle. Die antimikrobielle Wirkung der Glasoberfläche beruht ebenfalls auf dem Vorhandensein von antimikrobiell wirkenden Ionen. Weiterhin ist aber auch bekannt, dass Oberflächenladungen, d. h. das Zetapotential von Pulvern eine antimikrobielle Wirkung insbesondere auf Gram negative Bakterien haben kann. So geht von positiven Oberflächenladungen auf Gram negative Bakterien eine antimikrobielle Wirkung aus, da positive Oberflächenladungen Bakterien anziehen, aber Gram negative Bakterien nicht auf Oberflächen mit positvem Zetapotential wachsen, d. h. sich mehren können. Diesbezüglich wird auf Bart Gottenbos et al. Materials in Medicine 10 (1999) 853-855 Oberfläche von Polymeren verwiesen.

Antimikrobielle Effekte in Pulvern mit positiver Oberflächenladung werden in Speier et al. Journal of Colloid and Interface Science 89 68-76 (1982) Kenawy et al. Journal of controlled release 50, 145-52 (1998) beschrieben.

Durch Variation der glasbildenden, das heißt der netzwerkbildenden P₂O₅-Komponente kann die Lösegeschwindigkeit des Glases eingestellt werden. Durch den Ionenaustausch und die Auflösung des Glases wird die Freisetzungsrate biozider lonen eingestellt.

Insbesondere kann durch die Freisetzung von Phosphaten in wässriger Lösung der pH-Wert gezielt eingestellt werden, insbesondere auf einen hautneutralen Wert.

Durch den gezielten Einbau beispielsweise von Ti und/oder Zr wird des weiteren die Netzwerkbildung unterbrochen und die Reaktivität des Phosphatglases eingestellt, da eingebrachte biozid wirkende Ionen wie Zn, Ag leichter abgegeben werden können.

Zur Erzielung von gewünschten Farbwirkungen bspw. bei Anwendungen in Farben und Lacken können den Gläsern einzelne oder auch mehrere farbgebende Komponenten wie z.B. Fe₂O₃, CoO, CuO, V₂O₅, Cr₂O₅ in einer Gesamtkonzentration kleiner 4 Gew.-%, vorzugsweise kleiner 1 Gew.-% zugesetzt werden.
Die biozide beziehungsweise biostatische Wirkung des erfindungsgemäßen Glases beziehungsweise hieraus gewonnen Glaspulvers beziehungsweise der aus diesen Ausgangsgläsern gewonnen erfindungsgemäßen Glaskeramiken oder Glaskeramikpulver, wird durch lonenfreisetzung in einem flüssigen Medium, insbesondere in Wasser, verursacht. Die Gläser beziehungsweise die hieraus erhaltenen Glaspulver und Glaskeramiken weisen gegenüber Bakterien, Pilzen sowie Viren , Algen, Hefe eine biozide Wirkung auf.

Die Zugabe von Silber führt wie zuvor beschrieben sehr oft zu Verfärbungen des Glases. Eine derartige Verfärbung kann vermieden werden, wenn dem Glas Silber im Gemenge in oxidativ wirksamer Form, z.B. als Silbernitrat (AgNO₃) zugesetzt wird. Weiterhin wird das Glas bevorzugt unter oxidierenden Bedingungen, z.B. mittels Sauerstoff-Bubbling erschmolzen, um im Glas einen oxidierenden Zustand zu erreichen und somit eine Reduktion des Ag⁺ zu metallischem Ag ⁰ zu vermeiden. Dies kann auch durch Wanneneinstellungen erreicht werden wie z.B. durch oxidative Brennereinstellungen. Mit einer derartigen Verfahrensführung kann bei Zugabe von Silber eine Verfärbung sowohl im Glas als auch bei der Weiterverarbeitung im Polymer vermieden werden. Auch andere Komponenten wie z.B. Alkalien oder Erdalkalien können bevorzugt als oxidativ wirksame Komponenten zugesetzt werden, wie z.B. Nitrate, Peroxide etc. die dem Gemenge zugesetzt werden.

Im Vergleich zu den aus dem Stand der Technik bekannten silikatischen Gläser besitzen die hier beschriebenen Phosphatgläser eine höhere Reaktivität und somit eine bessere antimikrobielle Wirksamkeit. Weiterhin besitzen die hier beschriebenen Phosphatgläser eine niedrigere Glastemperatur (Tg) und können somit bei niedrigeren Temperaturen und damit leichter verarbeitet werden. Weiterhin kann es bei Mischungen der hier beschriebenen bei relativ niedrigen Temperaturen schmelzenden Gläser mit hochschmelzenden Polymeren zu einem teilweisen oder vollständigen Aufschmelzen der Gläser kommen, so dass die Gläser eine innigere Verbindung zum Polymer bilden, was bis zu einer extrem homogenen Verteilung im Polymer führen kann. Ein Aufschmelzen der Gläser wie beschrieben kann beispielsweise bei der Verarbeitung von erfindungsgemäßen Polymer-Glas-Kompositmaterialien zu Kunststoffhalbzeuge bzw. Kunststoffprodukte mit bioziden Eigenschaften erreicht werden. Diesbezüglich wird insbesondere auf das Aufschmelzen beim Extrudieren der Polymer-Glas-Kompositmaterialien verwiesen. Durch dieses Aufschmelzen wird die antimikrobielle Wirksamkeit erhöht sowie eine höhere Festigkeit des Polymer-Glas-Kompositmaterials erreicht. Weiterhin wird die Brennbarkeit bzw. Temperaturbeständigkeit des Materials erhöht. Bei den aus dem Stand der Technik, beispielsweise der PCT/EP03/00559 bekannten Silicatgläsern, die Kunststoffen zugemischt werden können, wird eine derartiges Aufschmelzen nicht beobachtet. Außerdem ist die antimikrobielle Wirkung von derartigen Mischungen deutlich geringer als bei Mischungen von Kunststoffen mit den erfindungsgemäßen Gläsern. Desweiteren lässt sich der Brechungsindex gezielt durch die Zugabe von beispielsweise Ti und/oder Zr einstellen.

Mit Hilfe von Mahlprozessen können die Glaszusammensetzungen zu Glaspulver mit Partikelgrößen < 100 µm gemahlen werden. Als zweckmäßig haben sich Partikelgrößen < 50 µm bzw. 20 µm erwiesen. Besonders geeignet sind Partikelgrößen < 10 µm sowie kleiner 5 µm. Als ganz besonders geeignet haben sich Partikelgrößen < 2 µm bzw. < 1 um herausgestellt.

Die Glaszusammensetzung bzw. hieraus gewonnene Glaskeramiken bzw. hieraus gewonnene Glaspulver bzw. Glaskeramikpulver, Glasfasern, Glasgranulate, Glaskugel können im Bereich der Kosmetik/Medizin eingesetzt werden , da sie in der Regel toxikologisch unbedenklich sind.

Die Ausführungsformen der Erfindung, die sich durch eine toxikologische Unbedenklichkeit auszeichnen ist besonders für eine Verwendung in Cremes bzw. Lotionen oder ähnlichen Darreichungsformen geeignet um sie auf die Haut aufzubringen.

Auf dem Gebiet der Medizin sind die Verringerung bzw. Vermeidung von Hautirritationen wie Hautrötung, Reizung sowie die Versorgung von Wunden im kosmetischen und medizinischen Bereich mögliche Anwendungen.

Ein weiteres Anwendungsfeld ist die Konservierung von Lebensmitteln.

Für Anwendungen in Bereichen, in denen das Glas, die hieraus gewonnene Glaskeramik oder das Glas- oder Glaskeramikpulver in Kontakt mit dem Menschen kommen, beispielsweise bei Anwendungen im Bereich der Medizin, der Kosmetik etc. ist das Glas bevorzugt frei von anderen Schwermetallen. Bei derartigen Anwendungen werden bevorzugt auch besonders reine Rohstoffe verwendet.

Die erfindungsgemäßen Gläser, Glaspulver, Glaskeramiken oder Glaskeramikpulver, Glasfasern, Glasgranulate, Glaskugeln können auch zur Konservierung der Produkte selbst sowie zur Erzielung einer antimikrobiellen Wirkung nach außen, d.h. einer Freisetzung von antimikrobiell wirksamen Substanzen, insbesondere Ionen wie z.B. Zink oder Silber verwendet werden.

Für die Verwendung der Glaszusammensetzungen bzw. Glaskeramiken bzw. Glaspulvern bzw. Glaskeramikpulvern, Glasfasern, Glasgranulate, Glaskugel zur Erzielung einer antimikrobiellen/bioziden Wirkung in Produkten beispielsweise in Farben und Lacken, ist die toxikologische Unbedenklichkeit keine Bedingung. Für derartige Verwendungen kann die Zusammensetzung Cr₂O₃ und/oder CuO enthalten.

Die erfindungsgemäßen der Glaszusammensetzungen bzw. Glaskeramiken bzw. Glas- oder Glaskeramikpulver, Glasfasern, Glasgranulate, Glaskugel können auf diesem Gebiet zur Konservierung der Produkte selbst und/oder zur Erzielung einer antimikrobiellen Wirkung nach außen, d.h. einer Freisetzung von antimikrobiell wirksamen Substanzen, insbesondere lonen wie z.B. Zink oder Silber verwendet werden.

Das Glas bzw. die Glaskeramik bzw. das Glas- oder Glaskeramikpulver Glasfasern, Glasgranulate, Glaskugel kann bei ausreichender hoher hydrolytischer Beständigkeit auch als Coating, d.h. Schutzschicht, auf ein Polymer aufgebracht werden.

Die biozide beziehungsweise biostatische Wirkung des erfindungsgemäßen Glases beziehungsweise hieraus gewonnen Glaspulvers beziehungsweise der aus diesen Ausgangsgläsern gewonnen erfindungsgemäßen Glaskeramiken, wird durch lonenfreisetzung in einem flüssigen Medium, insbesondere in Wasser, verursacht. Die Gläser beziehungsweise die hieraus erhaltenen Glaspulver und Glaskeramiken weisen gegenüber Bakterien, Pilzen, Algen sowie Viren eine biozide Wirkung auf. Diese Wirkung wird insbesondere durch das Vorhandensein von Silber und Zink verursacht.

Ein bevorzugtes Anwendungsfeld der Gläser oder der hieraus gewonnenen Glaskeramik, Glaspulver oder Glaskeramikpulver gemäß der Erfindung ist die Verwendung in Polymeren zur Erzielung einer bioziden bzw. biostatischen Wirkung. Zum einen kann eine Konservierung des Polymers selbst im Vordergrund stehen, d.h, das Polymer vor Bakterien und Pilzbefall zu schützen. Weiterhin kann hiermit eine biostatische bzw. biozide Polymeroberfläche geschaffen werden, wobei möglichst keine biozid wirksamen Stoffe, z.B. Ionen, an die Umgebung abgegeben werden sollen. Ein weiteres Ziel kann die Bereitstellung Polymeren sein, das insbesondere biozid wirksame Stoffe freisetzt.

In einem weiteren Aspekt der Erfindung wird daher ein Kunststoff-Glas-Kompositzur Verfügung gestellt, wobei das Kunststoff-Glas-Komposit-Material umfasst:
- ein Kunststoffmaterial
- ein Glas und/oder - eine Glaskeramik
basierend auf der Erfindungsgemäßen Glaszusammensetzung.

Für Glaszusammensetzungen in Polymeren wird erwartet, dass sie aufgrund der Abschirmung von wässrigen Medien nur ungenügend antimikrobiell sind, da sie Polymeren gekapselt werden. Überraschenderweise hat sich herausgestellt, das aber schon durch Zusatz von sehr geringen Mengen an bioziden lonen wie Ag, Zn, Cu, eine signifikante antimikrobielle Wirkung des Glases, der Glaskeramik, des Glaspulvers oder des Glaskeramikpulvers auftritt.

Dies ist deswegen überraschend, weil schon der sehr geringe Wassergehalt, in konventionell hergestellten Polymer ausreicht, um blozide Ionen in der Glasmatrix zu "aktivieren" und somit eine antimikrobielle Langzeitwirkung zu erzielen. Wird das Polymer-Glas- Komposit, das derartige Glaszusammensetzungen, Glaskeramiken, Glaspulver oder Glaskeramikpulver enthält, erwärmt, so kann das Glas je nach eingestellter Verarbeitungstemperatur teilweise aufschmelzen, wodurch die antimikrobielle Wirkung erhöht wird. Auch andere Eigenschaften des Komposit-Materials wie z. B. die Festigkeit werden positiv beeinflusst.

Wie zuvor ausgeführt, zeigen die Gläser mit den erfindungsgemäßen Zusammensetzungen beziehungsweise die hieraus gewonnenen Glaskeramiken, Glaspulver oder Glaskeramikpulver eine biostatische beziehungsweise biozide Wirkung in Polymeren. Diese kann dazu genutzt werden, Polymere zu konservieren, insbesondere vor Pilzbefall oder Zersetzung durch Bakterien zu Denkbar ist auch die Ausrüstung eines Polymeren mit einer antimikrobiellen Oberfläche. Bei einer solchen antimikrobiellen Oberfäche soll möglichst keine Freisetzung beziehungsweise Abgabe von antimikrobiell wirksamen Substanzen, insbesondere lonen nach außen, d.h. außerhalb der erfolgen.

Auch ermöglichen die erfindungsgemäßen Gläser eine langsame Freisetzung von antimikrobiell wirksamen Ionen aus einer Polymermatrix.
Hierbel spielt der Wassergehalt des Polymers sowie die Diffusion, der in der mobilen Ionen die entscheidende Rolle. Im Allgemeinen sind hier auch die Gehalte an bioziden Ionen in der Glasmatrix höher bzw. die des Glases im Polymer als in der oben genannten Anwendung. Diese Freisetzung kann verbunden sein mit einer teilweisen oder kompletten Auflösung des Glases. In einer besonders bevorzugten Ausführungsform löst sich auch die Polymermatrix teilweise oder vollständig auf. Dies ist insbesondere dann der Fall, wenn die Polymermatrix wasserlöslich ist.

Überraschenderweise hat sich herausgestellt, dass eine starke antimikrobielle Wirkung auch ohne das Vorhandensein von Alkalien in der Glasmatrix erzielt wird. Über den Gehalt an Alkaliionen wird gewöhnlich die Reaktivität des Glases eingestellt und somit die Stärke der antimikrobiellen Wirkung sowohl zeitlich als auch quantitativ. In den hier beschriebenen Gläser kann eine unterschiedliche Reaktivität auch mit einem geringen Gehalt bei weitgehend alkalifreien Glaszusammensetzungen eingestellt werden. Bei erfindungsgemäßen Gläsern werden durch Reaktionen an der Oberfläche des Glases Erdalkalien des Glases durch H⁺-Ionen des wässrigen Mediums ausgetauscht.

In einer Ausführungsform der Erfindung ist der Glaszusatz im Polymer selbst enthalten. In einer alternativen Ausgestaltung der Erfindung kann der Glaszusatz auch beispielsweise mit einem Precursor-Material als Coating oder Schutzschicht auf das Polymer aufgebracht werden kann.

Aus den hier beschriebenen Gläsern können Glaskeramiken bzw. Keramiken erhalten werden. Diese werden durch einen nachgeschalteten Temperschritt entweder am Halbzeug (bspw. den Glasbändern oder Ribbons) oder am Produkt, beispielsweise am Glaspulver oder den Glasfasern hergestellt. Im Anschluss an den Temperschritt kann eine erneute Mahlung notwendig sein, um die gewünschte Partikelgröße einzustellen.
Je nach Partikelgröße, Konzentration und der Zusammensetzung des Pulvers werden pH-Werte mit einer Untergrenze von < 1,0, bevorzugt < 1,5, ganz besonders bevorzugt < 2. Die Obergrenze beträgt < 9,0 und 8,0 , bevorzugt 7,5 und 7,0 und ganz besonders bevorzugt < 6.

Der Mahlprozess kann sowohl trocken als auch mit nichtwässrigen bzw. wässrigen Mahlmedien durchgeführt werden.

Mischungen verschiedener Glaspulver aus dem Zusammensetzungsberelch mit unterschiedlichen Zusammensetzungen und Korngrößen sind möglich, um bestimmten Effekte zu kombinieren.

Mischungen von Pulver mit unterschiedlicher Partikelgrösse können dazu verwendet werden, um eine starke und kurzeitige antimokrobielle Wirkung mit einer Langzeit- antimikrobiellen Wirkung zu kombinieren.

Eine bevorzugte Ausführungsform umfasst ein Mischung von zwei oder mehr Pulvern, wobei ein Pulver gemäß dem beschriebenen Zusammensetzungsbereich eine Korngrößenverteilung d50 von 8-500µm, bevorzugt 10-100µm, ganz besonders bevorzugt 10-30 um, ein anderes Pulver eine Korngrößenverteilung d50 von 0,01-100µm, bevorzugt 0,1- 20 µm ganz besonders bevorzugt 0,5 - 2 µm.

Eine weitere bevorzugte Ausführungsform umfasst eine Mischung von zwei oder mehreren Pulvern, wobei die Verhältnisse der spezifischen Oberflächen des einen Pulvers zum anderen Pulver der Mischung 0,001 zu 100 bevorzugt 0,01 zu 50 besonders bevorzugt 0,1 zu 10.

Mischungen von Glaspulvern mit unterschiedlichen Zusammensetzungen und Korngrößen können zur Einstellung spezieller Eigenschaften der einzelnen Glaspulver synergistisch kombiniert werden. So ist es beispielsweise möglich, die antimikrobielle Wirkung des Glaspulvers durch die Partikelgröße zu steuern.

Neben dem direkten Einbringen in die Glasmatrix während des Schmelzprozesses können diese lonen auch über einen Ionenaustausch nur in die Oberflächenbereiche des Glases eingebracht werden.

Das erfindungsgemäße Glas ruft in einer besonders bevorzugten Ausführungsform keine hautirritierenden Wirkungen hervor.

Durch eine Kombination der pH-Wirkung, der Wirkung durch Oberflächeneffekte und der Ag, Cu oder Zn-Abgabe kann eine erhebliche Steigerung der antimikrobiellen Wirkung erzielt werden, die über die Summe der Einzelwirkungen deutlich hinausgeht. Die in das Produkt freigesetzte Konzentration von Ag, Cu, Zn-Ionen kann hierbei deutlich unter 1 ppm liegen.

Die Einbringung des Ag, Cu, Zn kann hierbei entweder bereits bei der Schmelze durch entsprechende Salze erfolgen oder aber durch Ionenaustausch des Glases nach der Schmelze,

Gläser, Glaspulver, Glaskeramiken oder Glaskeramikpulver mit innerhalb des beanspruchten Zusammensetzungsbereiches liegender Zusammensetzung erfüllen alle Anforderungen bezüglich eines Einsatzes in den Bereichen Papierhygiene, Kosmetik, Farben, Lacken. Polymere, Putzen, Medizinprodukten, kosmetischen Anwendungen, Nahrungsmittelzusatz sowie Verwendung in Deoprodukten, Anti-Transpiranten sowie in Produkten zur Behandlung von Hautirritationen, akuten und chronischen Wunden sowie im Bereich der Zahnpflege/Zahnhygiene und Mundpflege/Mundhygiene sowie als Dentalmaterialien beispielsweise für Zahnfüllungen, Kronen, Inlets, etc.

Das Glas, die Glaskeramik, das Glas- Glaskeramikpulver kann in jeder geeigneten Form eingesetzt werden. Mischungen unterschiedlicher Glaspulver aus dem Zusammensetzungsbereich mit unterschiedlichen Zusammensetzungen sind ebenfalls möglich. Die Mischung mit anderen Glaspulvern ist ebenfalls möglich, um bestimmte Effekte zu kombinieren.

Komponenten wie Fluor können je nach Anwendungsgebiet dem Glas bis zu Konzentrationen von in Summe 5 Gew.-% zugesetzt werden. Diese Ausführungsform findet besonders im Bereich der Zahnpflege und Zahnhygiene Anwendung, da neben der antimikrobiellen und entzündungshemmenden Wirkung durch diese Ausführungsform Fluor in geringen Konzentrationen freigesetzt werden kann, das den Zahnschmelz härtet.

Eine besonders bevorzugte Anwendung ist die Verwendung der beschriebenen Gläser für Dentalmaterialien. Insbesondere für Zahnfüllungen, Kronen, Inlets, Besonders bevorzugt ist hierbei die Verwendung als Compositwerkstoff mit Polymerwerkstoffen.

Ohne den Einsatz der beschriebenen alkalifreien Gläsern im Polymerbereich einzuschränken, gibt es Polymere, die sich besonders zur Zugabe von der hier beschriebenen Gläser eignen. Dies sind insbesondere PMMA; PVC; PTFE; Polystyrol; Polyacrylat; Polyethylen; Polyester; Polycarbonat; PGA bioabbaubares Polymer; LGA bioabbaubares Polymer oder die Biopolymere Kollagen; Fibrin; Chitin; Chitosan; Polyamide; Polycarbonate; Polyester; Polyimide; Polyharnstoff; Polyurethane; Organische Fluoropolymere; Polyacrylamide und Polyacrylsäuren; Polyacrylate; Polymethacrylate; Polyolefine; Polystyrene und Styren-Copolymere; Polyvinylester; Polyvinylether; Polyvinylidenchlorid; Vinylpolymere; Polyoxymethylen; Polyaziridine; Polyoxyalkylene; Synthetische Harze bzw. Alkyl-Harze, Amino-Harze, Epoxy-Harze, Phenolische-Harze oder ungesättigte Polyester-Harze; elektrisch leitende Polymere; Hochtemperatur-Polymere; anorganische Polymere; Polyphenyloxid-Silicone; Biopolymere wie beispielsweise Cellulose, Cellulose-Ester, Cellulose-Ether, Enzyme, Gelatine, natürliche Harze, Nukleinsäuren , Polysaccharide, Proteine, Seide, Stärke oder Wolle.

Besonders bevorzugt ist die Verwendung der hier beschriebenen Gläser für Polymere, die bekanntermaßen eine Alkali-Unverträglichkeit zeigen können, wie beispielsweise Polycarbonate, bei denen es durch die Alkaliionen zu Kettenbrüche kommen kann.

Insbesondere eignen sie sich für die Verwendung in folgenden Produkten, beispielsweise als antimikrobieller Zusatz in Polymeren :
Schneidbrettern
Handschuhe
Mülleimer
Messergriffe
Essbesteck, beispielsweise Chopsticks
Tabletts
Tischdecken

Kühlschränken
Spülmaschinen
Wäschetrocknern
Waschmaschinen
Telefone
Tastaturen
Bügeleisen
Reiskocher

Lenkräder
Autoamaturen
Armlehnen
Schlüssel
Türgriff
Ascher
Schaltgriff
Schalter

Kugelschreiber
Disketten
Audio- Video-Kasetten
Compact Disks (CD)
Cilpboards

Des weiteren können derartige Gläser, Glaskeramiken, Glaspulver oder auch Glaskeramikpulver auch im Bereich der Bekleidungsindustrie, vorzugsweise als Zusatz zu Kunstfasern, Verwendung finden. Ein Einsatz in
Kleidungsstücken
Socken
Unterwäsche
Handtüchern
Toilettentüchern
Tapeten
Kissenbezügen
Kissenfüllungen
Badekleidung
Bademützen

ist denkbar.

Weitere Produkte auf Kunstfaser- oder Polymerbasis die das erfindungsgemäße Glas , die erfindungsgemäße Glaskeramik, ein hieraus gewonnenes Glas- oder Glaskeramikpulver enthalten können sind:
Teppichböden
Kontaktlinsen
Kontaktlinsenhalter- Gefässe
Spielsand
Plastikgeld
Papiergeld
Spielzeug
Armbanduhr
Taucherkleidung

Insbesondere für die Verwendung in Fasern für Teppichböden ist das antimikrobielle Glaspulver als Zumischung zu den Fasern besonders geeignet.

Das in dieser Erfindung beschriebene Glas beziehungsweise die hieraus gewonnene Glaskeramik oder das hieraus gewonnene Glas- oder Glaskeramikpulver, das durch Mahlen erhalten wird, ist wasserlöslich, aber verfügt über ausreichende chemische Beständigkeit. Das Glas beziehungsweise Glaspulver wirkt in erster Linie durch Ionenaustausch bzw. Ionenabgabe, was mit einer Oberflächenreaktion, und Metallionen-Freisetzung verbunden ist.

Überraschenderweise zeigen die Glas- oder Glaskeramikpulver gemäß der Erfindung eine hohe Reaktivität und einen höheren antimikrobiellen Effekt als die Gruppe der bioaktiven, alkalifreien Gläser, die im Stand der Technik beschrieben wurden, oder Glaspulvern, die aus derartigen Gläsern hergestellt wurden.

Die Erfindung soll nachfolgend anhand der Ausführungsbeispiele sowie der Figuren beschrieben werden.

Es zeigen:

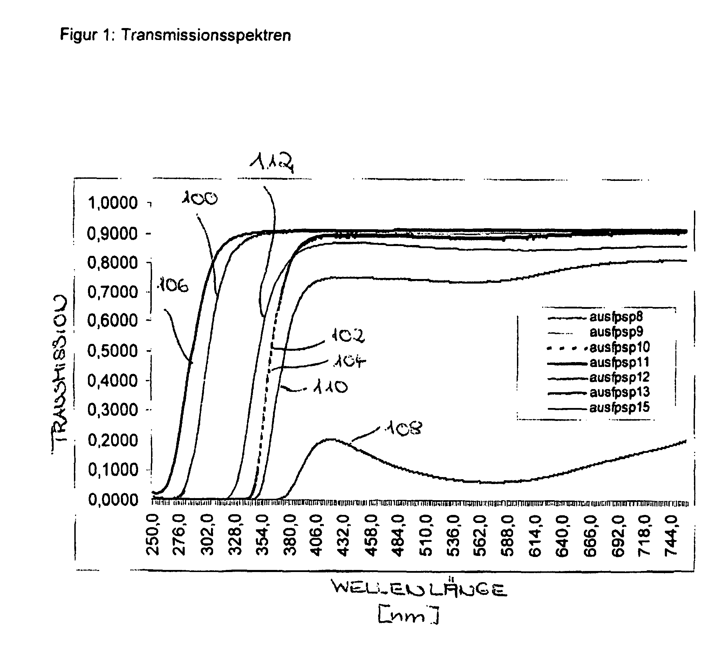

Figur 1: Transmissionsspektren für verschiedene Glaspulver

Die beschriebenen Gläser können neben dem konventionellen Schmelzprozeß auch über Sol-Gel Verfahren hergestellt werden.

Das Glas wurde aus den Rohstoffen in einem Kieselglas-Tiegel erschmolzen, und anschließend zu Ribbons verarbeitet. Die Ribbons wurden mittels Trockenmahlung zu Pulver mit einer Partikelgröße d50 = 4 µm weiterverarbeitet. Als Schmelztiegel können auch Platintiegel, Quarzaltiegel oder ZAC-Tiegel (ZAC: Baddelyit-Korund-Steine) dienen. Bei ZAC handelt es sich um schmelzgegossenes Zirkoniumaluminiumoxid. Quarzaltiegel sind Tiegel aus gesintertem Quarzglaspulver oder Quarzglasgries, wobei das gesinterte Quarzglaspulver oder der Quarzglaskies porös sind.

In Tabelle 1 werden die Zusammensetzungen und Eigenschaften von alkalifreien Gläsern angegeben, die zu den erfindungsgemäßen Glaspulvern gemahlen werden können. Die Zusammensetzungen beziehen sich auf Synthesewerte in Gew.-% auf Oxidbasis.

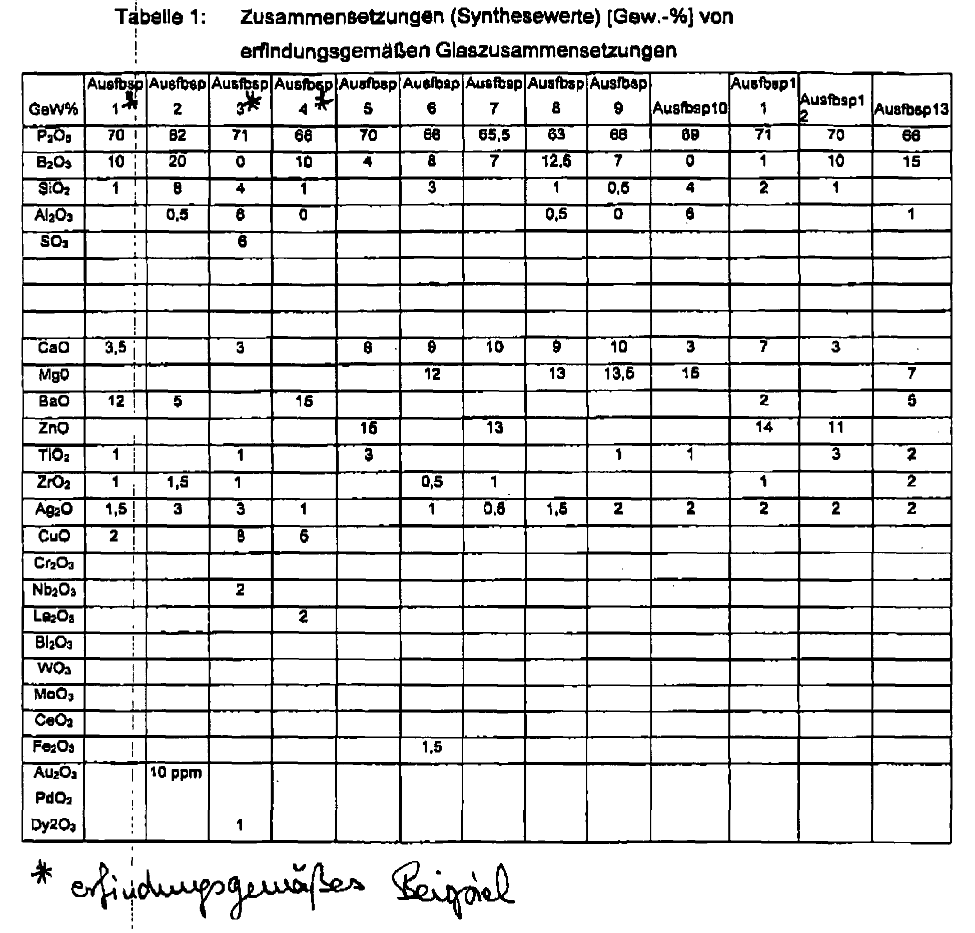

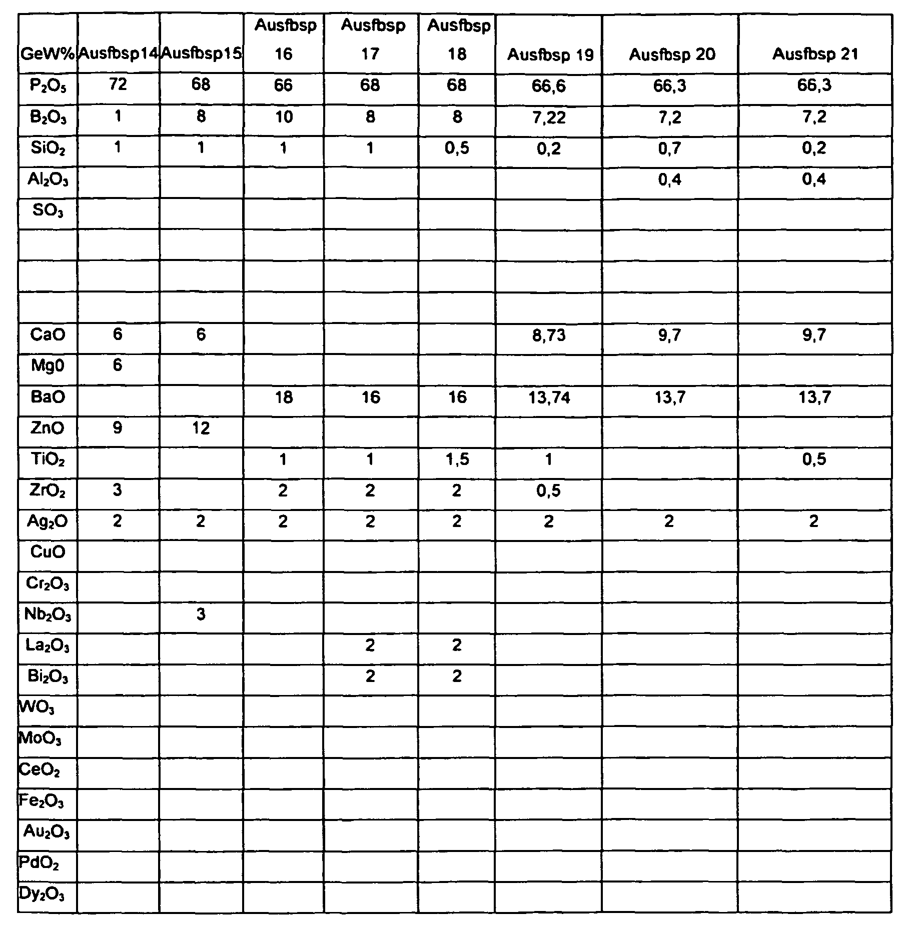

Nachfolgend sollen für einige der oben genannten Ausführungsbeispiele die pH-Werte, Leitfähigkeiten, der Brechungsindex, das Transmissionsverhalten, die Rate der Ag-lonen-Freisetzung sowie die chemische Beständigkeit angegeben werden.

In Tabelle 2 sind die pH-Werte und die Leitfähigkeit von 1 Gew.-% Glaspulver der Glaszusammensetzungen nach Ausführungsbeispielen 11-15 in Tabelle 1 in wässriger Lösung angegeben:

**Tabelle 2: pH Werte und Leitfähigkeit Werte, 1-Gew.-% Pulver in wässeride Lösung**

| | nach 15 min | | nach 60 min. | | nach 24 Stunden | |
|---|---|---|---|---|---|---|
| | pH-Wert | Leitfähigkeit | pH-Wert | Leitfähigkeit | pH-Wert | Leitfähigkeit |
| | | [µS/cm] | | [µS/cm] | | [µS/cm] |
| Ausfbsp 11 | 2,65 | 726 | 2,52 | 1011 | 2,03 | 2740 |
| Ausfbsp 12 | 2,70 | 998 | 2,24 | 1290 | 1,97 | 2700 |
| Ausfbsp 13 | 2,71 | 639 | 2,45 | 872 | 2,19 | 2170 |
| Ausfbsp 14 | 2,64 | 692 | 2,40 | 1049 | 2,10 | 2880 |
| Ausfbsp 15 | 2,39 | 1285 | 2,14 | 1726 | 1,78 | 4530 |

In Tabelle 3 ist als Maß für die chemische Beständigkeit der Gewichtsverlust von 1 Gew.-% Glaspulver einer Glaszusammensetzung gemäß einem der Ausführungsbeispiele 12 bis 16 in wässriger Lösung bei Raumtemperatur (RT) in Abhängigkeit von der Zeit angegeben:

**Tabelle 3: Chemische Beständigkeit (Gewichtverlust von 1 Gew.-% Glaspulver in wässerige Lösung bei Raum Temperatur (RT)**

| | Gewichtsverlust [%] | |
|---|---|---|
| | 1 h, RT | 24 h, RT |
| Ausfbsp 12 | 7,3 | 27,9 |
| Ausfbsp 13 | 8,3 | 22,4 |
| Ausfbsp 14 | 7,1 | 22,6 |
| Ausfbsp 15 | 15,9 | 49,4 |
| Ausfbsp 16 | 11,6 | 37,3 |

In Tabelle 4 ist die Silberionenfreisetzung (von 1 Gew.-% Glaspulver einer Glaszusammensetzung gemäß einem der Ausführungsbeispiele 12 bis 16 angegeben.

**Tabelle 4: Silberionenfreisetzung**

| | 1 Std | 24 Std |
|---|---|---|
| Ausfbsp 12 | 17,4 mg/L | 54,7 mg/L |
| Ausfbsp 13 | 16,1 mg/L | 43,7 mg/l |
| Ausfbsp 14 | 14,8 mg/L | 46,0 mg/L |
| Ausfbsp 15 | 30,5 mg/L | 94,9 mg/L |
| Ausfbsp 16 | 23,1 mg/L | 73,1 mg/L |

In Tabelle 5 ist die antibakterielle Wirkung eines Glaspulvers nach Europ. Pharmakopoe (3. Auflage) für eine Glaszusammensetzung gemäß Ausführungsbeispiel 9 in Tabelle 1 mit einer Partikelgröße von 4 µm in einer wässrigen Suspension bei einer Konzentration von 0,01 Gew.-% angegeben. Das Glas wurde vor der Mahlung nicht getempert. Hierbei bezeichnet der jeweilige Startwert die Anzahl der Bakterien zu Beginn der Versuche und die folgenden Werte die Anzahl der Bakterien nach der angegebenen Zeit. Hierbei bezeichnet ein Wert von 0, dass keinerlei Bakterien mehr vorlagen, womit die antimikrobielle Wirkung des Glaspulvers belegt ist.

**Tabelle 5: Antibakterielle Wirkung eines Glaspulvers in wässriger Lösung gemäß Ausführungsbeispiel 9**

| | E. coli | P. aeruginosa | S. aureus | C. albicans | A. niger |
|---|---|---|---|---|---|
| Start | 350000 | 250000 | 270000 | 333000 | 240000 |
| 2 Tage | 0 | 0 | 0 | 0 | 100 |
| 7 Tage | 0 | 0 | 0 | 0 | 100 |
| 14 Tage | 0 | 0 | 0 | 0 | 0 |
| 21 Tage | 0 | 0 | 0 | 0 | 0 |
| 28 Tage | 0 | 0 | 0 | 0 | 0 |

In Tabelle 6 sind die Brechungsindizes nD für die Glaspulver gemäß Ausführungsbeispiel 9 bis Ausführungsbeispiel 21 in Tabelle 1 angegeben. Wie aus Tabelle 6 hervorgeht, kann durch die Zugabe von beispielsweise TiO₂ und/oder ZrO₂ und/oder BaO und/oder La₂O₃, und/oder Cr₂O₃ , und/oder Nb₂O₃ der Brechungsindex eingestellt werden kann.

**Tabelle 6 Brechungsindex**

| | Ausfbsp 9 | Ausfbsp 10 | Ausfbsp 11 | Ausfbsp 12 | Ausfbsp 13 | Ausfbsp 15 | Ausfbsp 16 | Ausfbsp 17 | Ausfbsp 18 | Ausfbsp 19 | Ausfbsp 20 | Ausfbsp 21 |
|---|---|---|---|---|---|---|---|---|---|---|---|---|
| nD | 1,55 | 1,51 | 1,53 | 1,55 | 1,55 | 1,54 | 1,56 | 1,56 | 1,57 | 1,58 | 1,57 | 1,58 |

Wie aus obiger Tabelle hervorgeht, liegen die Brechungsindizes nD der Glaspulver, die aus den Glaszusammensetzungen in Tabelle 1 erhalten wurden zwischen 1,51 und 1,59 und damit im Bereich der Brechungsindizes von Polymeren. Beispielsweise beträgt der Brechungsindex von Polystyrol nD Polysterol = 1,59, der von Polycarbonat nD Polycarbonat = 1,58, der von Polyester nD Polyester = 1,57, der von Polyamid nD Polyamide =1,53.

Mit Hilfe der erfindungsgemäßen Glaszusammensetzungen ist es also möglich ein antimikrobielles Glaspulver zur Verfügung zu stellen, dessen Brechungsindex an den des Polymeren angepasst ist, so dass eine Trübung weitgehend vermieden wird und eine hohe Transparenz gegeben ist, da beispielsweise Streueffekte an Partikeln mit unterschiedlichen Brechungsindizes vermieden werden.

Wie die Transmissionsspektren in Figur 1 für unterschiedliche Glaszusammensetzungen in Tabelle 1 zeigen, ist es möglich durch den gezielten Einsatz von UV-blockenden Ionen wie z.B. Ti die UV-Kante zu ändern.

Durch diese UV-Blockung kann die Vergilbung und/ oder die Sprödigkeit aufgrund von UV-Strahlung in Polymeren verringert und sogar ganz verhindert werden.

In Figur 1 ist das Transmissionsspektrum für Ausführungsbeispiel 8 mit 100 bezeichnet, für Ausführungsbeispiel 9 mit 102, für Ausführungsbeispiel 10 mit 104, für Ausführungsbeispiel 11 mit 106, für Ausführungsbeispiel 12 mit 108, für Ausführungsbeispiel 13 mit 110 und für Ausführungsbeispiel 15 mit 112.
Die Transmission ist an Festkörperprobe gemessen, wobei die Dicke der Proben 8 mm beträgt.

Wie aus nachfolgender Tabelle 7 hervorgeht erfüllt eine Probe mit 0,5 Gew.-% eines Glaspulvers gemäß Ausführungsbeispiel 10 (Tabelle 1) in Polytyrol den ASTM-E2180-1 Test für eine antimikrobielle Wirkung. Der getestete Keim ist Escherichia Coli

**Tabelle 7: ASTM-E 2180-1**

| | | Keime Anzahl der nach 0 Std auf der Polymerprobe : Start Wert | Anzahl der Keime nach 24 Std auf der Polymerprobe |
|---|---|---|---|
| 0,5 Gew.-% | | 1,5 E+06 | 1,0E02 |
| Glaspulver gem. Ausf. 10 in Polysterol | | | |

Ausf. 10: Reduzierung um 4 logarithmische Stufen: starke bakterizide/fungizide Wirkung (nach 24 stunden)

Auswertung nach 24 Stunden:
<1 log Stufe (Zehnerpotenz): keine signifikante bakterizide/fungizide Wirkung
>1 Zehnerpotenz <2 Zehnerpotenz: geringe bakterizide/fungizide Wirkung
>2 Zehnerpotenz <3 Zehnerpotenz: signifikante bakterizide/fungizide Wirkung
>3 log Stufe (Zehnerpotenz): starke bakterizide/fungizide Wirkung

## Patentansprüche

1. Antimikrobiell wirkende Phosphatglaszusammensetzung die nachfolgenden Komponenten in Gew.-% auf Oxidbasis umfasst:
| | |
|---|---|
| P₂O₆ | > 45 - 90 Gew.-% |
| B₂O₃ | zwischen 1 und 45 Gew.-% |
| SiO₂ | 0 - 40 Gew.-% |
| Al₂O₃ | 0 - 20 Gew.-% |
| SO₃ | 0 - 30 Gew.-% |
| Li₂O | 0 - 0,1 Gew.-% |
| Na₂O | 0 - 0,1 Gew.-% |
| K₂O | 0 - 0,1 Gew.-% |
| CaO | 0 - 40 Gew.-% |
| MgO | 0 - 40 Gew.-% |
| SrO | 0 - 15 Gew.-% |
| BaO | 0 - 40 Gew.-% |
| ZnO | 0 - 40 Gew.-% |
| Ag₂O | 0 - 5 Gew.-% |
| CuO | 0 - 15 Gew.-% |
| Cr₂O₃ | 0 - 10 Gew.-% |
| J | 0 - 10 Gew.-% |
| TeO₂ | 0 - 10 Gew.-% |
| GeO₂ | 0 - 10 Gew.-% |
| TiO₂ | 0 - 10 Gew,-% |
| ZrO₂ | 0 - 10 Gew.-% |
| La₂O₃ | 0 - 10 Gew,-% |
| Nb₂O₃ | 0 - 5 Gew,-% |
| CeO₂ | 0 - 5 Gew.-% |
| Fe₂O₃ | 0 - 5 Gew.-% |
| WO₃ | 0 - 5 Gew.-% |
| Bi₂O₃ | 0 - 5 Gew.-% |
| MoO₃ | 0 - 5 Gew.-% |
wobei die Summe aus TiO₂ und ZrO₂- und BaO und La₂O₃ und Cr₂O₃ und Nb₂O₃ im Bereich 0,01- 40 Gew.-%
und die Summe aus Ag₂O und ZnO und CuO und Cr₂O₃ und J und TeO₂ und GeO2 im Bereich 0,1 - 40 Gew.-% liegt und
die Zusammensetzung bis auf Verunreinigungen frei von Sn ist,
wobei Ag₂O im Bereich 0,1 Gew.-% < Ag₂O ≤ 3 Gew.-% liegt und der CuO-Gehalt im Bereich 0,1 Gew.-% < CuO ≤ 10 Gew.-% liegt.

2. Antimikrobiell wirkende Phosphatglas-Zusammensetzung nach Anspruch 1, **dadurch gekennzeichnet, dass**
die Summe aus Ag₂O und ZnO und CuO und Cr₂O₃ und J und TeO₂ und GeO₂ im Bereich 0,5 - 40 Gew.-% liegt.

3. Antimikrobiell wirkende Phosphatglas-Zusammensetzung nach einem der Ansprüche 1 bis 2, **dadurch gekennzeichnet, dass** die Summe aus Ag₂O und ZnO und CuO und Cr₂O₃ und J und TeO₂ und GeO₂ im Bereich 2,0 - 40 Gew.-% liegt.

4. Antimikrobielle Phosphatglas-Zusammensetzung nach einem der Ansprüche 1 bis 3, **dadurch gekennzeichnet, dass** Ag₂O im Bereich 0,3 Gew.-% < Ag₂O ≤ 2 Gew.-% liegt.

5. Antimikrobielle Phosphatglas-Zusammensetzung nach einem der Ansprüche 1 bis 4, **dadurch gekennzeichnet, dass** der ZnO-Gehalt > 5 Gew.-%, bevorzugt > 10 Gew.-% , noch bevorzugter > 20 Gew.-% ist.

6. Antimikrobielle Phosphatglas-Zusammensetzung nach einem der Ansprüche 1 bis 5, **dadurch gekennzeichnet, dass** der CuO-Gehalt im Bereich 0,5 Gew.-% < CuO ≤ 8 Gew.-% liegt.

7. Antimikrobielle Phosphatglas-Zusammensetzung nach einem der Ansprüche 1 bis 6, **dadurch gekennzeichnet, dass** die Summe der Cu-Gehalt und der Silber-Gehalt im Bereich 0,1 Gew.-% < CuO + Ag₂O ≤ 10 Gew.-%, besonders bevorzugt 0,3 Gew.-% < CuO + Ag₂O ≤ 8 Gew.-% liegt.

8. Antimikrobielle Phosphatglas-Zusammensetzung nach einem der Ansprüche 1 bis 7, **dadurch gekennzeichnet, dass** der Cr-Gehalt im Bereich 0,3 Gew.-% < Cr₂O₃ ≤ 7 Gew.-%, besonders bevorzugt 3 Gew.-% < Cr₂O₃ ≤ 5 Gew.-% liegt.

9. Antimikrobielle Phosphatglas-Zusammensetzung nach einem der Ansprüche 1 bis 8, **dadurch gekennzeichnet, dass** der Gehalt von Al₂O₃ geringer als 1 Gew.-% ist.

10. Antimikrobielle Phosphatglas-Zusammensetzung nach einem der Ansprüche 1 bis 9, **dadurch gekennzeichnet, dass** die Summe aus MgO + CaO + SrO + BaO zwischen 2 Gew.-% und 40 Gew.-% liegt.

11. Antimikrobiell wirkende Phosphatglas-Zusammensetzung gemäß einem der Ansprüche 1 bis 10, **dadurch gekennzeichnet, dass** die Summe aus Na₂O und K ₂O und Li₂O im Bereich 0-0,3 Gew.-% liegt.

12. Antimikrobielle Phosphatglas-Zusammensetzung nach einem der Ansprüche 1 bis 11, **dadurch gekennzeichnet, dass** die Summe der Gehalte aus TiO₂ + CeO₂ + Fe₂O₃ + WO₃ + Bi₂O₃ + MoO₃ + Nb₂O₃ kleiner 20 Gew.-%, bevorzugt kleiner 15 Gew.-%, noch bevorzugter kleiner 10 Gew.-% und größer 0,1 Gew.-% ist.

13. Antimikrobiell wirkende Phosphatglaskeramik, **dadurch gekennzeichnet, dass** Glaskeramik aus einem Ausgangsglas mit einer Glaszusammensetzung gemäß einem der Ansprüche 1 bis 12 erhalten wurde.

14. Antimikrobiell wirkendes Glas- oder Glaskeramikpulver, **dadurch gekennzeichnet, dass** das Glaspulver ein Glas mit einer Glaszusammensetzung gemäß einem der Ansprüche 1 bis 12 oder das Glaskeramikpulver eine Glaskeramik gemäß Anspruch 13 umfasst.

15. Antimikrobiell wirkendes Glas- oder Glaskeramikpulver nach Anspruch 14, **dadurch gekennzeichnet, dass** die Größe der Glas- oder Glaskeramikpartikel im Mittel < 20 µm, insbesondere < 10 µm ist.

16. Antimikrobiell wirkendes Glas- oder Glaskeramikpulver nach Anspruch 14, **dadurch gekennzeichnet, dass** die Größe der Glas- oder Glaskeramikpartikel im Mittel < 5 µm ist.

17. Antimikrobiell wirkendes Glas- oder Glaskeramikpulver nach Anspruch 14, **dadurch gekennzeichnet, dass** die Größe der Glas- oder Glaskeramikpartikel im Mittel < 1 µm ist.

18. Antimikrobleller Kunststoff-Glas-Kompositwerkstoff, umfassend ein Polymer sowie ein Glas und/oder eine Glaskeramik mit einer Zusammensetzung des Glases und/oder des Ausgangsglases der Glaskeramik gemäß einem der Ansprüche 1 bis 13.

19. Antimikrobieller Kunststoff-Glas-Kompositwerkstoff, umfassend ein Polymer sowie ein Glas- und/oder Glaskeramikpulver gemäß einem der Ansprüche 15 bis 17.

20. Antimikrobieller Kunststoff-Glas-Kompositwerkstoff nach Ansprüche 18 oder 19, **dadurch gekennzeichnet, dass** das Polymer ein thermoplastischer, duoplastischer oder elastomerer Kunststoff ist.

21. Antimikrobieller Kunststoff-Glas-Kompositwerkstoff nach einem der Ansprüche 18 bis 20, **dadurch gekennzeichnet, dass** das Polymer ausgewählt aus einem der folgenden Polymere ist: Polystyrol, Acrylonitril-Butadien-Styrene (ABS), Polycarbonat.

22. Verfahren zur Herstellung einer antimikrobiellen-Phosphatglas-Zusammensetzung gemäß einem der Ansprüche 1 bis 12, umfassend folgende Schritte: sämtliche Komponenten der Glaszusammensetzung, einschließlich Ag₂O, ZnO, und andere antimikrobiell wirkender Komponenten werden gemischt - ein antimikrobielles Glas oder Glaskeramik wird in einem Tiegel erschmolzen.

23. Verfahren gemäß Anspruch 22, **dadurch gekennzeichnet, dass** der Tiegel ein Platintiegel, Quarzal- oder ZAC-Tiegel ist.

24. Verfahren gemäß einem der Ansprüche 23 bis 24 **dadurch gekennzeichnet, dass** das erschmolzene Glas und/oder die erschmolzene Glaskeramik zu Glassträngen sogenannten Ribons umgeformt wird.

25. Verfahren zur Herstellung eines Kunststoff-Glas-Kompositenmaterial umfassend die folgenden Schritte:
- ein Polymer wird mit einem Glaspulver, das eine antimikrobiell wirkende Phosphatglas-Zusammensetzung gemäß einem der Ansprüche 1 bis 12 aufweist, gemischt, ergebend eine Polymer/Glaspulver-Mischung
- die Polymer/Glaspulver-Mischung wird in einem Mischer auf eine Temperatur im Bereich + 50°C bis + 350°C erwärmt.
- ergebend einen Kunststoff-Glas-Komposit-Werkstoff.

26. Verfahren nach Anspruch 25, **dadurch gekennzeichnet, dass** die PolymerGlaspulver-Mischung auf eine Temperatur im Bereich + 50°C bis + 350°C erwärmt wird.

27. Verfahren zur Herstellung eines Kunststoff-Glas-Komposit-Materials Granulats, umfassend die folgenden Schritte:
- Herstellen eines Kunststoff-Glas-Komposit-Material gemäß einem der Ansprüche 25 oder 26, das eine antimikrobiell wirkende Phosphatglas-Zusammensetzung gemäß einem der Ansprüche 1 bis 12 aufweist,
- Mahlen des Polymer-Glas-Komposit-Werkstoffes zu einem Granulat.

28. Verfahren zur Herstellung eines Kunststoffhalbzeuges oder eines Kunststoffendproduktes mit folgenden Schritten:
- Herstellen eines Kunststoff-Glas-Komposit-Material gemäß einem der Ansprüche 25 bis 26, das eine antimikrobiell wirkende Phosphatglas-Zusammensetzung gemäß einem der Ansprüche 1 bis 12 aufweist,
- Weiterverarbeitung des Kunststoff Glas-Komposit-Materials zu einem Kunststoffhalbzeug oder zu einem Kunststoffendprodukt.

29. Antimikrobiell wirkendes Glas oder Glaspulver oder Glaskeramik oder Glaskeramikpulver nach einem der Ansprüche 1 bis 17 zur Verwendung in Kosmetikprodukten.

30. Antimikrobiell wirkendes Glas oder Glaspulver oder Glaskeramik oder Glaskeramikpulver oder Polymer-Glas-Kompositwerkstoff nach einem der Ansprüche 1 bis 21 zur Verwendung in Deodorantprodukten, in medizinischen Produkten und Präparaten, in Kunststoffprodukten oder Kunststoffhalbzeugen, im Bereich der Papierhygiene, in Nahrungsmitteln, in Reinigungsmitteln, in Farben und Lacken, in Putzen, Zementen und Beton, in Produkten der Mundhygiene, Zahnpflege, Mundpflege und Zahnfleischpflege.

## Claims

1. An antimicrobial acting phosphate glass composition, comprising the following components in percent by weight on the oxide basis:
| | |
|---|---|
| P₂O₅ | > 45 to 90% by weight |
| B₂O₃ | between 1 and 45% by weight |
| SiO₂ | 0 to 40% by weight |
| Al₂O₃ | 0 to 20% by weight |
| SO₃ | 0 to 30% by weight |
| Li₂O | 0 to 0.1 % by weight |
| Na₂O | 0 to 0.1 % by weight |
| K₂O | 0 to 0.1 % by weight |
| CaO | 0 to 40% by weight |
| MgO | 0 to 40% by weight |
| SrO | 0 to 15% by weight |
| BaO | 0 to 40% by weight |
| ZnO | 0 to 40% by weight |
| Ag₂O | 0 to 5% by weight |
| CuO | 0 to 15% by weight |
| Cr₂O₃ | 0 to 10% by weight |
| J | 0 to 10% by weight |
| TeO₂ | 0 to 10% by weight |
| GeO₂ | 0 to 10% by weight |
| Ti=O₂ | 0 to 10% by weight |
| ZrO₂ | 0 to 10% by weight |
| La₂O₃ | 0 to 10% by weight |
| Nb₂O₃ | 0 to 5% by weight |
| CeO₂ | 0 to 5% by weight |
| Fe₂O₃ | 0 to 5% by weight |
| WO₃ | 0 to 5% by weight |
| Bi₂O₃ | 0 to 5% by weight |
| MoO₃ 0 | to 5% by weight, |
with the sum total of TiO₂ and ZrO₂- and BaO and La₂O₃ and Cr₂O₃ and Nb₂O₃ being in the range of 0.01 to 40% by weight, and the sum total of Ag₂O and ZnO CuO and Cr₂O₃ and J and TeO₂ and GeO₂ being in the range of 0.1 to 40% by weight, and the composition being free from Sn apart from impurities, with Ag₂O being in the range of 0.1 % by weight < Ag₂O ≤ 3% by weight and the CuO content lying in the range of 0.1 % by weight < CuO ≤ 10% by weight.

2. An antimicrobial acting phosphate glass composition according to claim 1, **characterized in that** the sum total of Ag₂O and ZnO and CuO and Cr₂O₃ and J and TeO₂ and GeO₂ lie in the range of 0.5 to 40% by weight.

3. An antimicrobial acting phosphate glass composition according to one of the claims 1 to 2, **characterized in that** the sum total of Ag₂O and ZnO and CuO and Cr₂O₃ and J and TeO₂ and GeO₂ lie in the range of 2 to 40% by weight.

4. An antimicrobial phosphate glass composition according to one of the claims 1 to 3, **characterized in that** Ag₂O lies in the range of 0.3% by weight < Ag₂O ≤ 2% by weight.

5. An antimicrobial phosphate glass composition according to one of the claims 1 to 4, **characterized in that** the ZnO content is > 5% by weight, preferably > 10% by weight, more preferably > 20% by weight.

6. An antimicrobial phosphate glass composition according to one of the claims 1 to 5, **characterized in that** the CuO content is in the range of 0.5% by weight < CuO ≤ 8% by weight.

7. An antimicrobial phosphate glass composition according to one of the claims 1 to 6, **characterized in that** the sum total of the Cu content and the silver content lies in the range of 0.1 % by weight < CuO + Ag₂O ≤ 10% by weight, especially preferably 0.3% by weight < CuO + Ag₂O ≤ 8% by weight.

8. An antimicrobial phosphate glass composition according to one of the claims 1 to 7, **characterized in that** the Cr content lies in the range of 0.3% by weight < Cr₂O₃ ≤ 7% by weight, especially preferably 3% by weight < Cr₂O₃ ≤ 5% by weight.

9. An antimicrobial phosphate glass composition according to one of the claims 1 to 8, **characterized in that** the Al₂O₃ content is lower than 1 % by weight.

10. An antimicrobial phosphate glass composition according to one of the claims 1 to 9, **characterized in that** the sum total of MgO + CaO + SrO+ BaO lies between 2% by weight and 40% by weight.

11. An antimicrobial acting phosphate glass composition according to one of the claims 1 to 10, **characterized in that** the sum total of Na₂O and K₂O and Li₂O lies in the range of 0 to 0.3% by weight.

12. An antimicrobial phosphate glass composition according to one of the claims 1 to 11, **characterized in that** the sum total of the contents of Ti₂O + CeO₂ + Fe₂O₃ + WO₃ + Bi₂O₃ + MoO₃ + Nb₂O₃ is lower than 20% by weight, preferably lower than 15% by weight, more preferably lower than 10% by weight and larger than 0.1 % by weight.

13. Antimicrobial acting phosphate glass-ceramics, **characterized in that** the glass-ceramics were obtained from a starting glass with a glass composition according to one of the claims 1 to 12.

14. An antimicrobial acting glass or glass-ceramics powder, **characterized in that** the glass powder comprises a glass with a glass composition according to one of the claims 1 to 12 or the glass-ceramics powder comprises a glass-ceramics material according to claim 13.

15. An antimicrobial acting glass or glass-ceramics powder according to claim 14, **characterized in that** the size of the glass or glass-ceramics particles is on average < 20 µm, especially < 10 µm.

16. An antimicrobial acting glass or glass-ceramics powder according to claim 14, **characterized in that** the size of the glass or glass-ceramics particles is on average < 5 µm.

17. An antimicrobial acting glass or glass-ceramics powder according to claim 14, **characterized in that** the size of the glass or glass-ceramics particles is on average <,1 µm.

18. An antimicrobial plastic/glass composite material, comprising a polymer and a glass and/or glass-ceramics with a composition of the glass and/or the starting glass of the glass-ceramics according to one of the claims 1 to 13.

19. An antimicrobial plastic/glass composite material, comprising a polymer and a glass and/or glass-ceramics powder according to one of the claims 15 to 17.

20. An antimicrobial plastic/glass composite material according to claim 18 or 19, **characterized in that** the polymer is a thermoplastic, duoplastic or elastomeric plastic.

21. An antimicrobial plastic/glass composite material according to one of the claims 18 to 20, **characterized in that** the polymer is chosen from one of the following polymers: polystyrene, acrylonitrile butadiene styrene (ABS), polycarbonate.

22. A method for producing an antimicrobial phosphate glass composition according to one of the claims 1 to 12, comprising the following steps: all components of the glass composition, including AG₂O, ZnO and other antimicrobial acting components, are mixed, an antimicrobial glass or glass-ceramics is molten in a crucible.

23. A method according to claim 22, **characterized in that** the crucible is a platinum crucible, quarzal or ZAC crucible.

24. A method according to one of the claims 23 to 24, **characterized in that** the molten glass and/or the molten glass-ceramics are formed into strands of glass, so-called ribbons.

25. A method for producing a plastic/glass composite material, comprising the following steps:
- a polymer is mixed with a glass powder which comprises an antimicrobial acting phosphate glass composition according to one of the claims 1 to 12, resulting in a polymer/glass powder mixture;
- the polymer/glass power mixture is heated in a mixer to a temperature in the range of +50°C to +350°C;
- resulting in a plastic/glass composite material.

26. A method according to claim 25, **characterized in that** the polymer/glass powder mixture is heated to a temperature in the range of +50°C to +350°C.

27. A method for producing a plastic/glass composite material granulate, comprising the following steps:
- production of a plastic/glass composite material according to one of the claims 25 or 26 which comprises an antimicrobial acting phosphate glass composition according to one of the claims 1 to 12;
- grinding of the polymer/glass composite material into a granulate.

28. A method for producing a semi-finished plastic product or a final plastic product, comprising the following steps:
- production of a plastic/glass composite material according to one of the claims 25 to 26 which comprises an antimicrobial acting phosphate glass composition according to one of the claims 1 to 12;
- further processing of the plastic/glass composite material into a semi-finished plastic product or into a final plastic product.

29. An antimicrobial acting glass or glass powder or glass-ceramics or glass-ceramics powder according to one of the claims 1 to 17 for use in cosmetic products.

30. An antimicrobial acting glass or glass powder or glass-ceramics or glass-ceramics powder according to one of the claims 1 to 21 for use in deodorant products, in medical products and preparations, in plastic products or semi-finished plastic products, in the area of paper hygiene, in foodstuffs, in detergents, in paints and dyes, in plaster, cement and concrete, in products of oral hygiene, dental care, mouth care and gum care.

## Revendications

1. Composition de verre au phosphate ayant une action antimicrobienne, qui contient les composants suivants en pourcentage du poids sur la base des oxydes :
| | |
|---|---|
| P₂O₅ | > 45-90 % en poids |
| B₂O₃ | entre 1 et 45 % en poids |
| SiO₂ | 0-40 % en poids |
| Al₂O₃ | 0-20 % en poids |
| SO₃ | 0-30 % en poids |
| Li₂O | 0-0,1 % en poids |
| Na₂O | 0-0,1 % en poids |
| K₂O | 0-0,1 % en poids |
| CaO | 0-40 % en poids |
| MgO | 0-40 % en poids |
| SrO | 0-15 % en poids |
| BaO | 0-40 % en poids |
| ZnO | 0-40 % en poids |
| Ag₂O | 0-5 % en poids |
| CuO | 0-15 % en poids |
| Cr₂O₃ | 0-10 % en poids |
| I | 0-10% en poids |
| TeO₂ | 0-10 % en poids |
| GeO₂ | 0-10 % en poids |
| TiO₂ | 0 -10 % en poids |
| ZrO₂ | 0-10 % en poids |
| La₂O₃ | 0-10 % en poids |
| Nb₂O₃ | 0-5 % en poids |
| CeO₂ | 0-5 % en poids |
| Fe₂O₃ | 0-5 % en poids |
| WO₃ | 0-5 % en poids |
| Bi₂O₃ | 0-5 % en poids |
| MoO₃ | 0-5 % en poids |
où la somme de TiO₂ et ZrO₂ et BaO et La₂O₃ et Cr₂O₃ et Nb₂O₃ est comprise entre 0,01 et 40 % en poids et la somme d'Ag₂O et ZnO et CuO et Cr₂O₃ et I et TeO₂ et GeO₂ est comprise entre 0,1 et 40 % en poids et
la composition ne contient pas d'étain, hormis les impuretés,
Ag₂O se situant dans une plage de 0,1 % en poids < Ag₂O ≤ 3 % en poids et la teneur en CuO se situant dans une plage de 0,1 % en poids < CuO ≤ 10 % en poids.

2. Composition de verre au phosphate ayant une action antimicrobienne selon la revendication 1, **caractérisée en ce que** la somme d'Ag₂O et ZnO et CuO et Cr₂O₃ et I et TeO₂ et GeO₂ se situe dans une plage de 0,5 à 40 % en poids.

3. Composition de verre au phosphate ayant une action antimicrobienne selon l'une des revendications 1 à 2, **caractérisée en ce que** la somme d'Ag₂O et ZnO et CuO et Cr₂O₃ et I et TeO₂ et GeO₂ se situe dans une plage de 2,0 à 40 % en poids.

4. Composition de verre au phosphate ayant une action antimicrobienne selon l'une des revendications 1 à 3, **caractérisée en ce qu'**Ag₂O se situe dans une plage de 0,3 % en poids < Ag₂O ≤ 2 % en poids.

5. Composition de verre au phosphate ayant une action antimicrobienne selon l'une des revendications 1 à 4, **caractérisée en ce que** la teneur en ZnO est supérieure à 5 % en poids, de préférence supérieure à 10 % en poids et en particulier supérieure à 20 % en poids.

6. Composition de verre au phosphate ayant une action antimicrobienne selon l'une des revendications 1 à 5, **caractérisée en ce que** la teneur en CuO se situe dans la plage de 0,5 % en poids < CuO ≤ 8 % en poids.

7. Composition de verre au phosphate ayant une action antimicrobienne selon l'une des revendications 1 à 6, **caractérisée en ce que** la somme de la teneur en cuivre et de la teneur en argent se situe dans la plage de 0,1 % en poids < CuO + Ag₂O ≤ 10 % en poids, de préférence de 0,3 % en poids < CuO + Ag₂O ≤ 8 % en poids.

8. Composition de verre au phosphate ayant une action antimicrobienne selon l'une des revendications 1 à 7, **caractérisée en ce que** la teneur en chrome se situe dans la plage de 0,3 % en poids < Cr₂O₃ ≤ 7 % en poids, de préférence de 3 % en poids < Cr₂O₃ ≤ 5 % en poids.

9. Composition de verre au phosphate ayant une action antimicrobienne selon l'une des revendications 1 à 8, **caractérisée en ce que** la teneur en Al₂O₃ est inférieure à 1 % en poids.

10. Composition de verre au phosphate ayant une action antimicrobienne selon l'une des revendications 1 à 9, **caractérisée en ce que** la somme de MgO + CaO + SrO + BaO est comprise entre 2 % en poids et 40 % en poids.

11. Composition de verre au phosphate ayant une action antimicrobienne selon l'une des revendications 1 à 10, **caractérisée en ce que** la somme de Na₂O et K₂O et Li₂O se situe entre 0 et 0,3 % en poids.

12. Composition de verre au phosphate ayant une action antimicrobienne selon l'une des revendications 1 à 11, **caractérisée en ce que** la somme des teneurs en TiO₂ + CeO₂ + Fe₂O₃+ WO₃ + Bi₂O₃ + MoO₃ + Nb₂O₃ est inférieure à 20 % en poids, de préférence inférieure à 15 % en poids, et en particulier inférieure à 10 % en poids et supérieure à 0,1 % en poids.

13. Vitrocéramique au verre au phosphate ayant une action antimicrobienne, **caractérisée en ce que** la vitrocéramique est obtenue à partir d'un verre de départ ayant une composition de verre selon l'une des revendications 1 à 12.

14. Poudre de verre ou de vitrocéramique ayant une action antimicrobienne, **caractérisé en ce que** la poudre de verre contient un verre ayant une composition de verre selon l'une des revendications 1 à 12 ou la poudre vitrocéramique contient une vitrocéramique selon la revendication 13.

15. Poudre de verre ou de vitrocéramique ayant une action antimicrobienne selon la revendication 14, **caractérisée en ce que** la taille des particules de verre ou de vitrocéramique est en moyenne inférieure à 20 µm, en particulier inférieure à 10 µm.

16. Poudre de verre ou de vitrocéramique ayant une action antimicrobienne selon la revendication 14, **caractérisée en ce que** la taille des particules de verre ou de vitrocéramique est en moyenne inférieure à 5 µm.

17. Poudre de verre ou de vitrocéramique ayant une action antimicrobienne selon la revendication 14, **caractérisée en ce que** la taille des particules de verre ou de vitrocéramique est en moyenne inférieure à 1 µm.

18. Matériau composite antimicrobien à base de matériau synthétique et de verre, contenant
un polymère et
un verre et/ou une vitrocéramique ayant une composition du verre et/ou de la
vitrocéramique selon l'une des revendications 1 à 13.

19. Matériau composite antimicrobien à base de matériau synthétique et de verre, comprenant
un polymère et
une poudre de verre et/ ou de vitrocéramique selon l'une des revendications 15 à 17.

20. Matériau composite antimicrobien à base de matériau synthétique et de verre selon la revendication 18 ou 19, **caractérisé en ce que** le polymère est un matériau synthétique thermoplastique, thermodurcissable ou élastomère.

21. Matériau composite antimicrobien à base de matériau synthétique et de verre selon l'une des revendications 18 à 20, **caractérisé en ce que** le polymère est choisi parmi les polymères suivants : polystyrène, acrylonitrile-butadiène-styrène (ABS), polycarbonate.

22. Procédé pour la production d'une composition de verre au phosphate antimicrobienne selon l'une des revendications 1 à 12, comprenant les étapes de mélange de tous les composants de la composition de verre, y compris Ag₂O, ZnO et autres composants ayant une action antimicrobienne et fusion d'un verre ou d'une vitrocéramique antimicrobiens dans un creuset.

23. Procédé selon la revendication 22, **caractérisé en ce que** le creuset est un creuset en platine, en Quarzal ou en ZAC.

24. Procédé selon l'une des revendications 23 à 24, **caractérisé en ce que** le verre fondu et/ou la vitrocéramique fondue sont mis en forme pour obtenir des barres de verres appelées rubans.

25. Procédé pour la production d'un matériau composite de matériau synthétique et de verre, comprenant les étapes suivantes :
- mélange d'un polymère avec une poudre de verre contenant une composition de verre au phosphate ayant une activité antimicrobienne selon l'une des revendications 1 à 12, pour obtenir un mélange de polymère et de poudre de verre ;
- chauffage du mélange de polymère et de poudre de verre dans un mélangeur jusqu'à une température comprise entre +50°C et +350°C,
- pour obtenir un matériau composite de matériau de synthèse et de verre.

26. Procédé selon la revendication 25, **caractérisé en ce que** le mélange de polymère et de poudre de verre est chauffé jusqu'à une température comprise entre + 50°C et + 350°C.

27. Procédé pour la production d'un matériau composite de matériau synthétique et de verre, comprenant les étapes suivantes :
- production d'un matériau composite de matériau de synthèse et de verre selon l'une des revendications 25 ou 26 qui contient une composition de verre au phosphate ayant une activité antimicrobienne selon l'une des revendications 1 à 12;
- broyage du matériau composite de matériau synthétique et de verre pour obtenir des granulés.

28. Procédé pour la production d'un produit semi-fini en matériau de synthèse ou d'un produit fini en matériau de synthèse, comprenant les étapes suivantes :
- production d'un matériau composite de matériau de synthèse et de verre selon l'une des revendications 25 à 26, contenant une composition de verre au phosphate ayant une action antimicrobienne selon l'une des revendications 1 à 12;
- traitement du matériau composite de matériau de synthèse et de verre pour obtenir un produit semi-fini en matériau de synthèse ou un produit fini en matériau de synthèse.

29. Verre ou poudre de verre ou vitrocéramique ou poudre de vitrocéramique ayant une action antimicrobienne selon l'une des revendications 1 à 17 pour utilisation dans des produits cosmétiques.

30. Verre ou poudre de verre ou vitrocéramique ou poudre de vitrocéramique ou matériau composite de polymère et de verre ayant une action antimicrobienne selon l'une des revendications 1 à 21 pour une utilisation dans des produits déodorants, des produits et préparations médicaux, des matières plastiques et produits semi-finis en matière plastique, dans l'hygiène du papier, dans le domaine alimentaire, dans les détergents, les peintures et les vernis, dans les enduits, ciments et bétons, dans les produits d'hygiène bucco-dentaire, de soins dentaires, de soins oraux et de soins gingivaux.
